# EUROPÄISCHE PATENTSCHRIFT

(11) **EP 2 113 770 B1**
(45) Veröffentlichungstag und Bekanntmachung des Hinweises auf die Patenterteilung: **01.02.2012**
(21) Anmeldenummer: 08155462.8
(22) Anmeldetag: 30.04.2008
(51) Int. Cl.: G01N 33/00, G01N 30/84

(54) **Vorrichtung und Verfahren zur Reduzierung eines Ethanolgehaltes einer flüssigen Zubereitung**
Device and method to reduce the ethanol content of a liquid preparation
Dispositif et procédé de réduction d'une teneur en éthanol d'une préparation liquide

(43) Veröffentlichungstag der Anmeldung: 04.11.2009
(73) Patentinhaber: Symrise AG, 37603 Holzminden (DE)
(72) Erfinder: Roloff, Michael, 34399 Oberweser-Arenborn (DE); Peter, Regina, 33034 Brakel-Istrup (DE); Lütkenhaus, Matthias, 37170 Uslar (DE); Reichelt, Katharina, 83026 Rosenheim (DE)
(74) Vertreter: Eisenführ, Speiser & Partner

(56) Entgegenhaltungen:
- WO-A-2006/111476
- DE-A1- 3 941 533
- DE-A1- 19 707 150

## Beschreibung

### Gebiet der Erfindung

Die vorliegende Erfindung betrifft eine Vorrichtung und ein Verfahren zur Reduzierung eines Ethanolgehaltes einer flüssigen Zubereitung, Verwendung der Vorrichtung zu Herstellung eines flüssigen Geschmackstoffkonzentrates und Verfahren zu dessen sensorischen Bewertung.

### Stand der Technik

Aromen sind meistens komplexe Gemische einer Vielzahl von sensorisch wirksamen Substanzen, wie z.B. Pflanzenextrakte oder natürliche, naturidentische oder synthetische Aromastoffe. Aromakompositionen umfassend oder bestehend aus sensorisch wirksamen Substanzen enthalten zumindest zwei verschiedene Aromastoffe, die flüchtig (Geruchsstoffe) oder nichtflüchtig (Geschmacksstoffe) sein können. In der menschlichen Nase können flüchtige Geruchstoffe retronasal oder orthonasal wahrgenommen werden. Die geschmacklichen (gustatorischen) Eindrücke umami, salzig, sauer, süß und bitter werden durch Interaktion der Geschmacksstoffe mit den Geschmacksrezeptoren der Zunge wahrgenommen, weiterhin können hierbei auch weitere sensorische Eindrücke, beispielsweise prickelnde, kühlende, scharfe, brennende oder elektrisierende ("tingling") Effekte, wahrgenommen werden, die oftmals auf Grund von trigeminalen Reizungen entstehen. Der sensorische Gesamteindruck einer Aromakomposition wird grundsätzlich durch die Mengenverhältnisse der sensorisch wirksamen Stoffe einer Aromakomposition zueinander beeinflusst, wobei der sensorische Beitrag der einzelnen Aromastoffe entscheidend ist und nicht deren absolute Menge. In Lebensmitteln enthaltene sensorisch relevante Aromastoffe sind häufig nur in sehr geringen Mengen enthalten und vielfach noch nicht bekannt, obwohl durch diese Aromastoffe ein bedeutender geruchlicher und/oder geschmacklicher Beitrag, aufgrund ihrer hohen sensorischen Aktivität, geliefert wird. Ein wichtiges Bewertungskriterium ist daher der Aromawert, definiert als Quotient der Konzentration eines sensorisch wirksamen Aromastoffes zum geruchlichen beziehungsweise geschmacklichen Schwellenwert.

In der Aromenindustrie kommt heute eine Vielzahl unterschiedlicher sensorischer Bewertungsverfahren bzw. Vorrichtungen zur sensorischen Bewertung zum Einsatz. Die zur Bewertung verwendeten Proben werden hierbei oftmals sehr arbeits- und zeitintensiv vorbereitet. Leichtflüchtige Lösungsmittelanteile in Probenextrakten werden häufig durch Verwendung separater Verfahren reduziert. Probenmischungen werden durch zusätzliche Arbeitsschritte realisiert. Für den einzelnen Anwender ist es äußerst schwierig, bzw. nahezu unmöglich, die Bedienung der Vielzahl von verschiedenen Vorrichtungen und Verfahren zu beherrschen. Er beschränkt sich daher notgedrungen auf die bekannten und verfügbaren Vorrichtungen und Verfahren, was dazu führen kann, dass in bestimmten Fällen geeignete Vorrichtungen oder Verfahren nicht angewendet werden. In der Zeitschrift Chemie in unserer Zeit 2003, 37, 388-401, bzw. der darin zitierten Literatur, ist ein Übersichtsartikel zu diesem Thema veröffentlich worden.

Zur Geschmacks- und Geruchsstoffsuche sind bis heute keine High-throughput screening Verfahren zur sensorischen Bewertung der Prüfverbindungen bekannt, die zur schnellen Identifizierung neuer, sensorisch interessanter Verbindungen eingesetzt werden. Deshalb setzt die systematische Untersuchung eines Naturstoffextraktes heutzutage eine mehrstufige Trennung und Vorbehandlung zur Aufreinigung und Aufkonzentrierung der im Extrakt enthaltenen Einzelverbindungen voraus. Insbesondere die Probenvorbereitung zur sensorischen Bewertung in unterschiedlichen Testmedien nimmt hierbei sehr viel Zeit in Anspruch. Die Artefaktbildung, Instabilität oder Aufarbeitungsverluste bei der Isolierung/Vermischung von Geruch- und/oder Geschmackstoffen aus/mit komplexen Matrices kann aber auch zu geruchlichen bzw. geschmacklichen Fehlinformationen führen und stellt den Analytiker kontinuierlich vor neue Herausforderungen. Es sind deshalb eine Vielzahl von unterschiedlichen schonenden Extraktions- und Isolierungsverfahren entwickelt worden, die insbesondere individuell auf spezifische Aufgabenstellungen eingehen.

Zur flüssigchromatographischen Trennung von Aromakompositionen werden üblicherweise Elutionsmittel (mobile Phase) eingesetzt, die mit Wasser mischbar, jedoch gesundheitlich bedenklich oder giftig sind. Häufig verwendete Elutionsmittel sind Acetonitril, Methanol und Tetrahydrofuran sowie deren Mischungen mit wässrigen Puffern oder Wasser. Diese gesundheitlich bedenklichen Lösungsmittel müssen vor einer sensorischen Evaluierung entfernt werden. Durch Einsatz zeitintensiver, extraktiver oder thermischer Verfahren zur Entfernung der gesundheitlich bedenklicher Lösungsmittel kommt es häufig zu einer Änderung der Zusammensetzung der flüssigchromatographisch gewonnenen Fraktionen. Ursache hierfür sind thermische und/oder oxidative Einflüsse während der Behandlung. Die sensorischen Eigenschaften derartig behandelter Fraktionen unterscheiden sich häufig erheblich von den ursprünglichen Kompositionen. In der WO 2006/111476 ist bereits ein Verfahren zur Trennung von geruchlich und/oder geschmacklich wirksamen Komponenten mittels Hochtemperatur-Flüssigchromatographie (HTLC) sowie ein Verfahren zur direkten Verkostung der durch dieses Verfahren gewonnenen Fraktionen bekannt. Nachteilig an diesem Verfahren ist, dass das Eluat eines separierten Aromastoffes auf Grund seiner Verteilungsseigenschaften einen Ethanolgehalt von deutlich mehr als 15 Gew.-% bezogen auf das Gesamtgewicht des Eluats umfassen kann. Ein Ethanolgehalt von mehr als 15 Gew.-% bezogen auf das Gesamtgewicht des Eluats kann jedoch zum einen zu einer Beeinflussung der Geruchs- bzw. Geschmacksrezeptoren einer Testperson und damit zur Fehlinformation der sensorischen Eigenschaften führen und zum anderen kann ein Ethanolgehalt von mehr als 15 Gew.-% bei einer Verkostung von üblicherweise 5 ml pro Probe zu einer zeitweisen Bewusstseinsbeeinträchtigung der Testperson bei Verkostung mehrerer Proben führen, so dass lediglich wenige Verkostungen am Tag durchgeführt werden können.

In der Druckschrift wird darauf verwiesen, dass ein zu hoher Ethanolgehalt des verkosteten Eluats die sensorische Beurteilung negativ beeinflussen kann, und dass eine Ethanolreduzierung vor der Verkostung durch Membranseparation oder Adsorptionsmethoden erfolgen kann. Desweiteren wird zur Empfindlichkeitssteigerung der sensorischen Beurteilung vorgeschlagen, das Eluat in einem Gasstrom zu zerstäuben und optional das zerstäubte Eluat zu verdampfen.

In DE 3 941 533 A1 wird eine Schnittstelle zum Koppeln eines Flüssigkeitschromatographiesystems mit einem Fest- oder Gasphasendetektor beschrieben. Die erfin-dungsgemäße Schnittstelle umfasst einen Vernebler mit Thermospray- oder andere Sprühtechnik, gekoppelt an eine Verdampfungskammer und einen technischen Detektor, beispielsweise Massendetektor, wobei bereits im Vernebler die Temperatur auf mehr als 100 °C erhöht wird, um besonders kleine Aerosoltröpfchen herzustellen und damit eine große Oberfläche zu erreichen, sodass im anschließenden Verdampfungsschritt fast das gesamte Lösungsmittel verdampft und ein "trockenes" Aerosol umfassend das Inertgas und die interessierenden Teilchen an den Gasphasen- oder Festphasen-Detektor zur Analyse weitergeleitet wird. Nachteilig an diesem Verfahren ist, dass keine sensorische Bewertung hinsichtlich der geruchlichen oder geschmacklichen Eigenschaften der einzelnen Aromastoffe durchgeführt wird.

Es sind weiterhin Bewertungsverfahren durch elektronische Sensorsysteme ("*in vitro*") bekannt. Nachteilig hierbei ist die häufig fehlende Übertragbarkeit auf den Geschmacks- und/oder Geruchseindruck des Menschen.

Der vorliegenden Erfindung liegt deshalb die Aufgabe zu Grunde eine Vorrichtung und ein Verfahren zur Herstellung einer Geschmackstoffzubereitung umfassend oder bestehend aus ein oder mehreren Geschmacksstoffen, Wasser und Ethanol zur (i) zeitnahen ("on-line") oder synchronen sensorischen Bewertung durch eine Testperson ("in vivo") hinsichtlich des Geschmackes (Verkostung) bereitzustellen, wobei die hergestellte Geschmackstoffzubereitung (ii) ein geringes Risiko der Fehleinschätzung des Geschmackseindruckes auf Grund der Beeinträchtigung der Geschmacksrezeptoren der Zunge durch den Ethanolgehalt aufweist und/oder (iii) eine Verkostung von zwei, drei, vier, fünf und mehreren Proben an einem Tag erlaubt, ohne, dass eine zeitweise Bewusstseinsbeeinträchtigung für die Testperson auf Grund des Alkoholgehaltes hervorgerufen wird. Desweiteren ist es Aufgabe der vorliegenden Erfindung eine solche Geschmackstoffzubereitung schnell und einfach herzustellen, wobei die enthaltenen Geschmackstoffe bezüglich ihres geschmacklichen Eindruckes (im wesentlichen) nicht beeinträchtigt werden.

### Zusammenfassung der Erfindung

Die Aufgaben der vorliegenden Erfindung werden vollständig oder teilweise durch die erfindungsgemäßen Gegenstände gemäß der unabhängigen Ansprüche gelöst.

Dementsprechend wird die Aufgabe der vorliegenden Erfindung gelöst durch eine Vorrichtung (1) zum Reduzieren eines Ethanolgehaltes in einer flüssigen Zubereitung mit einer Vernebeleinrichtung (2), einer Mischkammer (3), einer Separationskammer (4) und einer Kondensationskammer (5), dadurch gekennzeichnet, dass
- die Vernebeleinrichtung (2) einen Flüssigkeitseinlass (21), einen Gaseinlass (22) und eine Verbindung zur Mischkammer (3) umfasst oder daraus besteht und die Vernebeleinrichtung (2) bei einer Temperatur ≤ 100 °C betrieben wird, so dass eine Flüssigkeit umfassend Wasser und Ethanol mit einem Ethanolgehalt von > 15 Gew.-% bezogen auf das Gesamtgewicht der Flüssigkeit mittels des Flüssigkeitseinlasses (21) und ein Inertgas mittels des Gaseinlasses (22) der Vernebeleinrichtung (2) zuführbar sind und die Vernebeleinrichtung (2) geeignet ist, die Flüssigkeit und das Inertgas zu einem Aerosol zu vernebeln und so in die Mischkammer (3) zuzuführen,
- die Mischkammer (3) eine Verdampfungseinrichtung (31) und eine Verbindung zur Separationskammer (4) umfasst oder daraus besteht, wobei die Mischkammer (3) nach der Vernebeleinrichtung (2) angeordnet und so damit verbunden ist, dass das mittels der Vernebeleinrichtung (2) gebildete Aerosol in die Mischkammer (3) zuführbar ist und die Verdampfungseinrichtung (31) geeignet ist, einen Teil oder das Ethanol des zugeführten Aerosols teilweise oder vollständig in die Gasphase zu überführen,
- die Separationskammer (4) einen Gasauslass (41) und eine Verbindung zur Kondensationskammer (5) umfasst oder daraus besteht, wobei die Separationskammer (4) nach der Mischkammer (3) angeordnet und so damit verbunden ist, dass das in die Gasphase überführte Ethanol und das verbleibende Aerosol in die Separationskammer (4) zuführbar sind und ein Teil oder die Ethanol-Gasphase aus der Vorrichtung durch den Gasauslass (41) entfernbar ist und
- die Kondensationskammer (5) einen Flüssigkeitsauslass (51) und eine Kondensationseinrichtung (52)umfasst oder daraus besteht, wobei die Kondensationskammer (5) nach der Separationskammer (4) angeordnet und so damit verbunden ist, dass das in der Separationskammer (4) nach teilweiser oder vollständiger Entfernung der Ethanol-Gasphase verbleibende Aerosol in die Kondensationskammer (5) zuführbar ist und die Kondensationseinrichtung (5) geeignet ist, das zugeführte Aerosol ganz oder teilweise zu kondensieren und das Kondensat mit einem Ethanolgehalt von ≤ 15 Gew.-% bezogen auf das Gesamtgewicht des Kondensats aus der Kondensationskammer (5) durch den Flüssigkeitsauslass (51) entfernbar ist, wobei die Separationskammer (4) einen größeren Querschnitt im Vergleich zu Mischkammer (3) umfasst.

Ein zweiter erfindungsgemäßer Gegenstand betrifft ein Verfahren zur Reduzierung eines Ethanolgehaltes in einer Geschmackstoffzubereitung umfassend oder bestehend aus ein oder mehreren Geschmackstoffen, Wasser und Ethanol umfassend oder bestehend aus den folgenden Schritten:
a) Bereitstellung einer flüssigen Geschmackstoffzubereitung, die ein oder mehrere Geschmackstoffe, Wasser und Ethanol mit einem Ethanolgehalt von > 15 Gew.-% bezogen auf das Gesamtgewicht der Geschmackstoffzubereitung umfasst oder daraus besteht, sowie eines Inertgases,
b) Zuführung der flüssigen Geschmackstoffzubereitung durch einen Flüssigkeitseinlass (21) und des Inertgases durch einen Gaszugang (22) in eine Vernebeleinrichtung (2) mittels der die Geschmackstoffzubereitung und das Inertgas zu einem Aerosol vernebelt werden,
c) Zuführung des in Schritt b) gebildeten Aerosols in eine mit der Vernebeleinrichtung (2) verbundenen Mischkammer (3) mit Verdampfungseinrichtung (31) und Überführung eines Teils des oder des gesamten Ethanolanteils des Aerosols mittels der Verdampfungseinrichtung (31) in eine Gasphase,
d) Zuführung des in Schritt c) gebildeten Aerosols in eine mit der Mischkammer (3) verbundenen Separationskammer (4) mit Gasauslass (41) und Entfernung eines Teils des oder des gesamten gasförmigen Ethanolanteils aus der Vorrichtung durch den Gasauslass (41),
e) Zuführung des in Schritt d) gebildeten Aerosols in eine mit der Separationskammer (4) verbundene Kondensationskammer (5) mit Flüssigkeitsauslass (51) und Kondensationseinrichtung (52) und Kondensation des Aerosols mittels der Kondensationseinrichtung (52) zu einem Geschmackstoffkonzentrat umfassend oder bestehend aus dem oder den Geschmackstoffen, Wasser und Ethanol mit einem Ethanolgehalt von ≤ 15 Gew.-% bezogen auf das Gesamtgewicht des Geschmackstoffkonzentrats und
f) Entfernung des in Schritt e) gebildeten Geschmackstoffkonzentrats aus der Kondensationskammer (5) durch den Flüssigkeitsauslass (51), wobei die Separationskammer (4) einen größeren Querschnitt im Vergleich zu Mischkammer (3) umfasst.

Ein dritter erfindungsgemäßer Gegenstand betrifft ein Verfahren zur sensorischen Bewertung von ein oder mehreren Geschmackstoffen umfassend oder bestehend aus den folgenden Schritten:
i. Herstellung eines flüssigen Geschmackstoffkonzentrates umfassend oder bestehend aus ein oder mehreren Geschmackstoffen, Wasser und Ethanol mit einem Ethanolgehalt von ≤ 15 Gew.-% bezogen auf das Gesamtgewicht des Geschmackstoffkonzentrats gemäß einem der Verfahren des zweiten erfindungsgemäßen Gegenstandes sowie der bevorzugten Ausgestaltungen und
ii. Zuführung des Geschmackstoffkonzentrates in den Mund einer Testperson zur sensorischen Bewertung.

Ein vierter erfindungsgemäßer Gegenstand betrifft eine Verwendung einer erfindungsgemäßen Vorrichtung zur Herstellung eines flüssigen Geschmackstoffkonzentrates umfassend oder bestehend aus ein oder mehreren Geschmackstoffen, Wasser und Ethanol mit einem Ethanolgehalt von ≤ 15 Gew.-% bezogen auf das Gesamtgewicht des Geschmackstoffkonzentrats.

Die Gewichtsprozentanteile (Gew.-%) in Bezug auf die erfindungsgemäßen Gegenstände sind jeweils auf das Gesamtgewicht der jeweiligen flüssigen Zubereitung bezogen.

Die erfindungsgemäßen Gegenstände umfassen ebenfalls die bevorzugten Ausgestaltungen, wie sie in der nachfolgenden Beschreibung, Ausführungsbeispielen, Figuren und abhängigen Ansprüchen dargestellt werden.

### Figurenbeschreibung:

Die Figuren zeigen sowohl einen schematischen Aufbau einer erfindungsgemäßen Vorrichtung (1) als auch die erfindungsgemäße Vorrichtung (1) in Verbindung mit den vor- und nachgeschalteten Systemen.

### Von den Figuren ist:

- Fig. 1:: einen schematischen Aufbau einer erfindungsgemäßen Vorrichtung (1).
- Fig. 2:: einen schematischen Aufbau einer erfindungsgemäßen Vorrichtung (1) gekoppelt an ein Flüssigchromatographiesystem (6).
- Fig. 3:: einen schematischen Aufbau einer erfindungsgemäßen Vorrichtung (1) gekoppelt an ein Probengefäß (61) enthaltend eine Geschmackstoffprobe.

### Bezugszeichenliste:

- (1): erfindungsgemäße Vorrichtung
- (2): Vernebeleinrichtung
- (21): Flüssigkeitseinlass Vernebeleinrichtung
- (22): Gaseinlass Vernebeleinrichtung
- (3): Mischkammer
- (31): Verdampfungseinrichtung
- (32): Innere Wand der Mischkammer
- (33): Äußere Wand der Mischkammer
- (34): Einlass für ein Medium zur Temperaturregelung
- (35): Auslass für ein Medium zur Temperaturregelung
- (36): Gaseinlass Mischkammer
- (4): Separationskammer
- (41): Gasauslass
- (411): Ende des Gasauslass im unteren Bereich innerhalb der Separationskammer
- (412): Ende des Gasauslass im Bereich außerhalb der Separationskammer/Vorrichtung
- (42): Verwirbelungseinrichtung
- (43): Wand der Separationskammer
- (5): Kondensationskammer
- (51): Flüssigkeitsauslass
- (52): Kondensationseinrichtung
- (53): Innere Wand der Mischkammer
- (54): Äußere Wand der Mischkammer
- (55): Einlass für ein Medium zur Temperaturregelung
- (56): Auslass für ein Medium zu Temperaturregelung
- (6): Flüssigkeitschromatographiesystem
- (61): Probengefäß mit Geschmackstoffprobe
- (62): Pumpe
- (7): Inertgasbehälter
- (8): Detektoreinrichtung

### Detaillierte Beschreibung der Erfindung

Der vorliegenden Erfindung liegt die Erkenntnis zu Grunde, dass mit der erfindungsgemäßen Vorrichtung ein flüssiges Geschmackstoffkonzentrat umfassend oder bestehend aus ein oder mehreren Geschmackstoffen, Wasser und Ethanol mit einem Ethanolgehalt von ≤ 15 Gew.-% bezogen auf das Gesamtgewicht des Geschmackstoffkonzentrates in einer einfachen und schnellen Weise hergestellt werden kann und so (i) eine zeitnahe ("on-line") oder synchrone sensorische Bewertung durch eine Testperson ("in vivo") erlaubt. Das erfindungsgemäß herstellbare Geschmackstoffkonzentrat weist zudem (ii) bei der in vivo sensorischen Bewertung ein geringes Risiko für eine Fehleinschätzung der sensorischen Eigenschaften des oder der Geschmackstoffe auf, da auf Grund des Ethanolgehaltes von ≤ 15 Gew.-% bezogen auf das Gesamtgewicht des Geschmackstoffkonzentrates die Geschmacksrezeptoren nicht (wesentlich) beeinträchtigt werden und (iii) ermöglicht bei einer in vivo sensorischen Bewertung eine Verkostung von zwei, drei, vier oder mehreren Proben (üblicherweise ≤ 5 ml/Probe) eines oder mehrerer Geschmackstoffkonzentrates an einem Tag, ohne das Bewusstsein der Testperson zeitweise zu beeinträchtigen.

Im Sinne der vorliegenden Erfindung umfasst der Begriff "Geschmackstoff" eine sensorisch wirksame Substanz, die einen Geschmackseindruck hervorruft, d.h. selbst vermittelt oder die geschmackliche Wahrnehmung einer anderen Substanz auf Grund eines Eigengeschmacks verändert, d.h. modifiziert oder verstärkt, wobei die Substanz einen Dampfdruck < 0,01 Pa bei 25° C aufweist.

Im Sinne der vorliegenden Erfindung bedeutet der Begriff "Geschmackstoffzubereitung" eine flüssige Zubreitung umfassend oder bestehend aus ein oder mehreren Geschmackstoffen, Wasser und Ethanol mit einem Ethanolgehalt von > 15 Gew.-% bezogen auf das Gesamtgewicht der Geschmackstoffzubereitung. Bevorzugt sind solche Geschmackstoffzubereitungen in denen der oder die Geschmackstoffe einen Anteil von ≤ 5 Gew.-%, bevorzugt ≤ 2,5 Gew.-%, weiter bevorzugt ≤ 1 Gew.-% und mindestens 10⁻¹² Gew.-% und die Wasser-Ethanol-Mischung einen Anteil von größer oder gleich 95 Gew.-%, bevorzugt größer oder gleich 97,5 Gew.-%, weiter bevorzugt größer oder gleich 99 Gew.-% jeweils bezogen auf das Gesamtgewicht der Geschmackstoffzubereitung aufweist. Der Ethanolgehalt in der Wasser-Ethanol-Mischung liegt vorzugsweise im Bereich von 16 bis 95 Gew.-%, weiter bevorzugt im Bereich von 25 bis 90 Gew.-%, besonders bevorzugt im Bereich von 40 bis 85 Gew.-%, ganz besonders bevorzugt im Bereich von 50 bis 80 Gew.-%, jeweils bezogen auf das Gesamtgewicht der Wasser-Ethanol-Mischung. Je höher der Anteil an Ethanol in der Wasser-Ethanol-Mischung der Geschmackstoffzubereitung ist, desto weniger fein muss die Geschmackstoffzubereitung, bei vergleichbarer Temperatur, vernebelt werden, um eine erfindungsgemäße Reduzierung des Ethanolgehaltes zu erhalten.

Die erfindungsgemäß zu verwendende Wasser-Ethanol-Mischung der erfindungsgemäßen Geschmackstoffzubereitung kann zusätzlich einen oder mehrere Bestandteile umfassen oder daraus bestehen, die ausgewählt werden aus der Gruppe bestehend aus Propylenglykol, Glycerin, Triacetin (Glycerintriacetat), physiologisch verträglichen Salzen (beispielsweise Natriumchlorid), physiologisch verträglichen Säuren (beispielsweise Phosphorsäure, Essigsäure), physiologisch verträglichen Puffersubstanzen (beispielsweise Natriumphosphate, Natriumacetat), Öle und Fette, wobei die Konzentration des oder der Bestandteile in der Wasser-Ethanol-Mischung zweckmäßigerweise jeweils so gewählt wird, dass die erfindungsgemäße Geschmackstoffzubereitung als Ganzes gesundheitlich unbedenklich bleibt.

Sofern die vorstehend bevorzugte Wasser-Ethanol-Mischung der erfindungsgemäßen Geschmackstoffzubereitung physiologisch verträgliche Salze (beispielsweise Natriumchlorid), physiologisch verträgliche Säuren (beispielsweise Phosphorsäure, Essigsäure) und/oder physiologisch verträgliche Puffersubstanzen (beispielsweise Natriumphosphate, Natriumacetat) umfasst, liegt der Gesamtanteil dieser Salze, Säuren und/oder Puffersubstanzen vorzugsweise bei ≤ 10 Gew.-%, bevorzugt bei ≤ 5 Gew.-%, besonders bevorzugt im Bereich von 0,001 bis 2 Gew.-%, jeweils bezogen auf das Gesamtgewicht der Geschmackstoffzubereitung.

Im Sinne der vorliegenden Erfindung wird das "Geschmackstoffkonzentrat" aus der erfindungsgemäßen Geschmackstoffzubereitung hergestellt und umfasst oder besteht aus ein oder mehreren Geschmackstoffen, Wasser und Ethanol mit einem Ethanolgehalt von ≤ 15 Gew.-%, bevorzugt im Bereich von 0 bis 10 Gew.-%, weiter bevorzugt im Bereich von 0,1 bis 8 Gew.-%, besonders bevorzugt im Bereich von 1 bis 5 Gew.-%, jeweils bezogen auf das Gesamtgewicht des Geschmackstoffkonzentrates. Dementsprechend liegt das erfindungsgemäße Geschmackstoffkonzentrat im Vergleich zur erfindungsgemäßen Geschmackstoffzubereitung in Bezug auf den Ethanolgehalt reduziert vor. Desweiteren können die zusätzlichen Bestandteile der Geschmackstoffzubereitung ebenfalls in dem erfindungsgemäßen Geschmackstoffkonzentrat vorliegen.

Im Sinne der vorliegenden Erfindung bedeutet "flüssige Zubereitung", die in die erfindungsgemäße Vorrichtung (1) zugeführt werden kann, eine flüssige Zubereitung umfassend Wasser und Ethanol mit einem Ethanolgehalt von > 15 Gew.-% bezogen auf das Gesamtgewicht der flüssigen Zubereitung. Der Ethanolgehalt in der flüssigen Zubereitung liegt vorzugsweise im Bereich von 16 bis 95 Gew.-%, weiter bevorzugt im Bereich von 25 bis 90 Gew.-%, besonders bevorzugt im Bereich von 40 bis 85 Gew.-%, ganz besonders bevorzugt im Bereich von 50 bis 80 Gew.-%, jeweils bezogen auf das Gesamtgewicht der flüssigen Zubereitung. In einer bevorzugten Ausgestaltung stellt die flüssige Zubereitung die erfindungsgemäße flüssige Geschmackstoffzubereitung dar. Das aus der "flüssigen Zubereitung" mittels der erfindungsgemäßen Vorrichtung zu erhaltende "Kondensat" umfasst oder besteht dementsprechend aus Wasser und Ethanol mit einem Ethanolgehalt von ≤ 15 Gew.-%, bevorzugt im Bereich von 0 bis 10 Gew.-%, weiter bevorzugt im Bereich von 0,1 bis 8 Gew.-%, besonders bevorzugt im Bereich von 1 bis 5 Gew.-%, jeweils bezogen auf das Gesamtgewicht des Kondensats. In einer bevorzugten Ausgestaltung stellt das erfindungsgemäße Kondensat das erfindungsgemäße Geschmackstoffkonzentrat dar.

Im Sinne der vorliegenden Erfindung bedeutet der Begriff "Inertgas", dass das zugeführte Inertgas die erfindungsgemäße "flüssigen Zubereitung", insbesondere die erfindungsgemäße "Geschmackstoffzubereitung" nicht in chemischer Hinsicht verändert. Inertgase werden bevorzugt ausgewählt aus der Gruppe bestehend aus: Stickstoff, Helium oder Argon. Besonders bevorzugt wird Stickstoff als Inertgas erfindungsgemäß verwendet.

Im Sinne der vorliegenden Erfindung bedeutet der Begriff "sensorische Bewertung", dass eine Probe, üblicherweise ≤ 5 ml, eines erfindungsgemäß hergestellten Geschmackstoffkonzentrates durch eine Testperson in den Mund genommen und gegebenenfalls geschluckt wird, wobei die Probe gustatorsich und/oder retronasal hinsichtlich der sensorischen Eindrücke, beispielsweise umami, salzig, sauer, süß, bitter, oder prickelnde, kühlende, scharfe, brennende oder elektrisierende ("tingling") Effekte, bewertet wird.

Im Sinne der vorliegenden Erfindung ist ein erfindungsgemäß zu verwendendes "Flüssigkeitschromatographiesystem (6)" eine Vorrichtung, die zur Durchführung einer Flüssigkeitschromatographie [liquid chromatography (LC-Chromatographie), bevorzugt high pressure liquid chromatography (HPLC-Chromatographie) und insbesondere high temperature liquid chromatography (HTLC-Chromatographie)] einer Geschmackstoffzubereitung mit Detektion des oder der Geschmackstoffe geeignet ist.

Geeignete Trenntechniken für die erfindungsgemäß zu verwendenden Flüssigkeitschromatographiesysteme (6) umfassen: Reversed-Phase Chromatographie (RP), Normal-Phase Chromatographie (NP), Größenausschluß-Chromatographie (SEC) und Hydrophile Interaktionschromatographie (HILIC).

Üblicherweise werden die Detektoren ausgewählt aus der Gruppe bestehend aus Massenselektiven Detektoren (MS), Ultraviolett Detektoren (UV), Diodenarray Detektoren (DAD), Brechungsindex-Detektoren (RI), Verdampfungslichtstreu Detektoren (ELSD - evaporative light scattering detector), Fluoreszenz Detektoren (FLD) oder Charged Aerosol Detektoren (CAD).

Bevorzugt umfasst oder besteht ein HPLC-Flüssigkeitschromatographiesystem (6) aus einem Degasser (beispielsweise 3315, Fa. ERC), zwei Pumpen (beispielsweise Sun Flow 100, Fa. Sunchrom), einem Mischer (beispielsweise dynamic/static Mixer, Fa. Sunchrom), einem Autosampler (beispielsweise Midas, Fa. Spark), einer Chromatographiesäule (beispielsweise einer Hamilton PRP-1 250x10mm Säule) und einem Detektor (bevorzugt einem Diodenarray-Detector, beispielsweise Spectra Flow 600, Fa. Sunchrom). Bei einem erfindungsgemäß zu verwendenden HTLC-Flüssigkeitschromatographiesystem ist die Chromatographiesäule üblicherweise in einem Säulenofen geeignet zur Einstellung der benötigten Temperatur in der Säule (beispielsweise Polaratherm, Fa. Selerity) angeordnet.

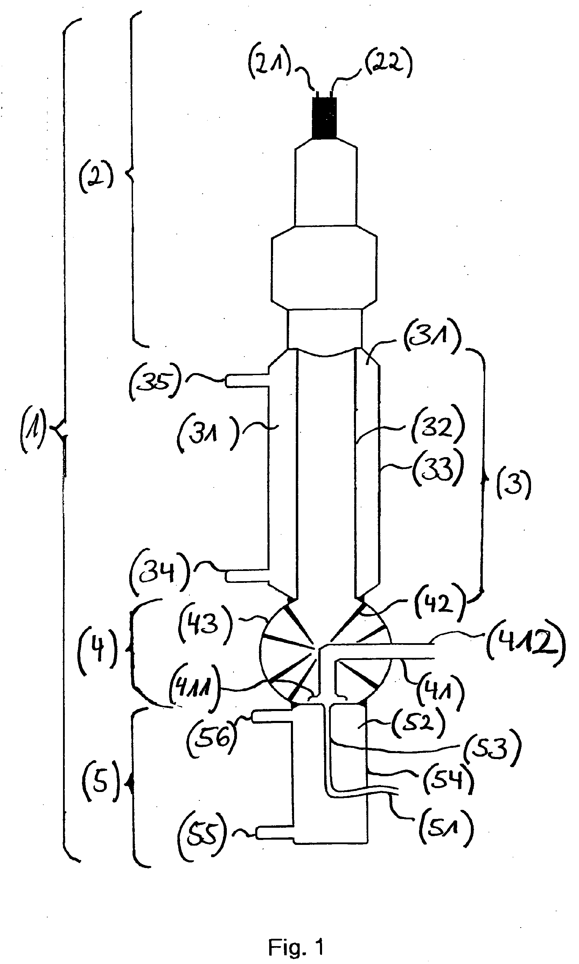

Figur 1 zeigt einen schematischen Aufbau (im Maßstab 1 : 2, d.h. die Vorrichtung ist doppelt so groß wie die Abbildung in Figur 1) einer erfindungsgemäßen Vorrichtung (1), die zur erfindungsgemäßen Reduzierung eines Ethanolgehaltes einer erfindungsgemäßen flüssigen Zubereitung, bevorzugt einer erfindungsgemäßen Geschmackstoffzubereitung, geeignet ist.

Die flüssige Zubereitung, bevorzugt Geschmackstoffzubereitung wird durch den Flüssigkeitseinlass (21) in die Vernebeleinrichtung (2) zugeführt. Zusätzlich wird ein Inertgas durch den Gaseinlass (22) (im wesentlichen) gleichzeitig zur Flüssigkeitseinleitung in die Vernebeleinrichtung (2) zugeführt. Mittels der Vernebeleinrichtung (2) wird die zugeführte Flüssigkeit und das zugeführte Inertgas zu einem Aerosol vernebelt und in die Mischkammer (3) als Aerosol zugeführt.

Erfindungsgemäß wird die Vernebeleinrichtung (2) bei einer Temperatur ≤ 100 °C, vorzugsweise ≤ 75 °C, weiter bevorzugt in einem Bereich von 10 bis 40 °C, besonders bevorzugt in einem Bereich von 15 bis 30 °C und ganz besonders bevorzugt in einem Bereich von 20 bis 25 °C betrieben. Dadurch, dass die Arbeitstemperatur in der Vernebeleinrichtung (2) im Bereich ≤ 100 °C erfindungsgemäß betrieben wird, wird beispielsweise das Risiko von Siedeverzügen des Wasser-Ethanol-Gemisches reduziert. Zudem kann man über die Einstellung der Temperatur die Tröpfchengröße des Aerosols regeln. Bei Tröpfchen des Aerosols mit einem durchschnittlich kleineren Durchmesser ist die Oberfläche der Flüssigkeit im Aerosol größer als bei Tröpfchen mit einem durchschnittlich größeren Durchmesser. Je größer die Oberfläche der Tröpfchen im Aerosol, desto höher wird der Anteil der Verdampfung des Ethanols bzw. des Ethanol-Wasser-Gemisches im Aerosol im anschließenden Verdampfungsschritt sein.

Die zugeführte Flüssigkeit und das zugeführte Inertgas werden mit einer üblichen Fließgeschwindigkeit bzw. einem üblichen Druck in die Vernebeleinrichtung zugeführt, damit einerseits die Vernebelung zu einem Aerosol ermöglicht wird und andererseits das gebildete Aerosol durch die erfindungsgemäße Vorrichtung (1) transportiert werden kann. Fließgeschwindigkeiten und Drücke können von der Ausgestaltung der Vernebeleinrichtung (2) abhängen und dementsprechend variieren.

Erfindungsgemäß zu verwendende Vernebeleinrichtungen (2) sind bevorzugt Vernebeleinrichtungen für Verdampfungslichtstreudetektoren (beispielsweise Evaporative Light Scattering Detector, ELSD), die beispielsweise in US 7,290,723, US 6,528018, US 6,485,689, US 6,362,880, US 6,229,605, US 6,151,113 und US 6,122,055 dargestellt werden, sowie Thermospray-Vernebler, die beispielsweise in DE 3 941 533 A1, US 4,730,111 und US 4,629,478 dargestellt werden. Besonders bevorzugt sind Vernebeleinrichtungen für Verdampfungslichtstreudetektoren, beispielsweise die Vernebeleinrichtung der Fa. Sedere für den Flußbereich von 1 - 5ml/min.

Die bevorzugte Vernebeleinrichtung der Fa. Sedere umfasst üblicherweise einen Gaseinlass (22) mit Regler zur Einstellung eines Arbeitsdrucks in der Vernebeleinrichtung (2) im Bereich von 0,5 bis 5 bar, vorzugsweise im Bereich von 1 bis 4 bar, weiter bevorzugt 2 bis 3 bar und ganz besonders bevorzugt bei 3 bar.

Zusätzlich oder alternativ umfasst der Flüssigkeitseinlass (21) üblicherweise einen Regler zur Einstellung der Fließgeschwindigkeit der Flüssigkeit in die Vernebeleinrichtung (2) im Bereich von 1 bis 5 ml/min, vorzugsweise, 2 bis 4 ml/min, besonders bevorzugt 3 ml/min.

Die vorstehend dargestellten bevorzugten Fließgeschwindigkeiten und Arbeitsdrücke ermöglichen eine besonders bevorzugte Vernebelung zu einem Aerosol und einen bevorzugten anschließenden Weitertransport des gebildeten Aerosols durch die erfindungsgemäße Vorrichtung (1).

In einer weiteren bevorzugten Ausgestaltung ist die erfindungsgemäße Vorrichtung (1) zusätzlich oder alternativ zu den vorstehenden bevorzugten Ausgestaltungen senkrecht zum Erdmittelpunkt aufgebaut, so dass die Vernebeleinrichtung (2) am oberen Ende, d.h. an dem zum Erdmittelpunkt entferntest gelegenen Ende, der erfindungsgemäßen Vorrichtung (1), und die Kondensationskammer (5) am unteren Ende, d.h. an dem zum Erdmittelpunkt am nächsten gelegenen Ende, der erfindungsgemäßen Vorrichtung (1) angeordnet. Dieser Aufbau ist vorteilhaft, da die Schwerkraft zusätzlich zum vorliegenden Arbeitsdruck zum Weitertransport des Aerosols durch die erfindungsgemäße Vorrichtung (1) beiträgt und damit die Fließgeschwindigkeit und/oder der Arbeitsdruck verringert werden können.

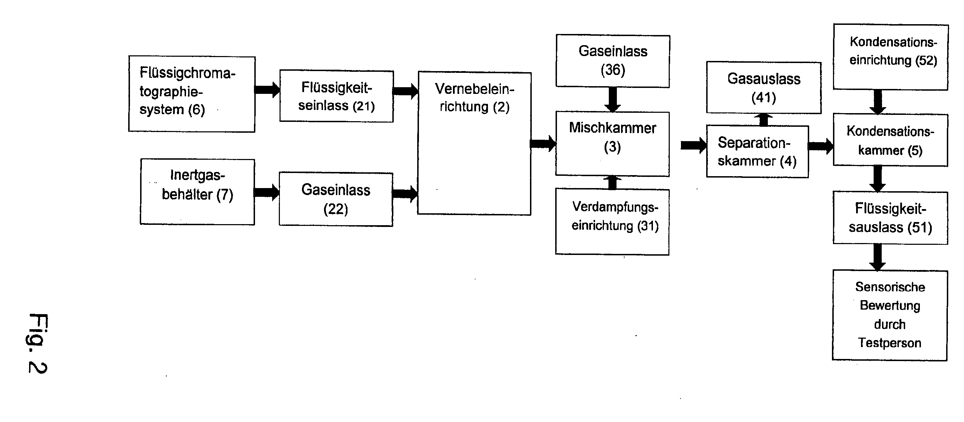

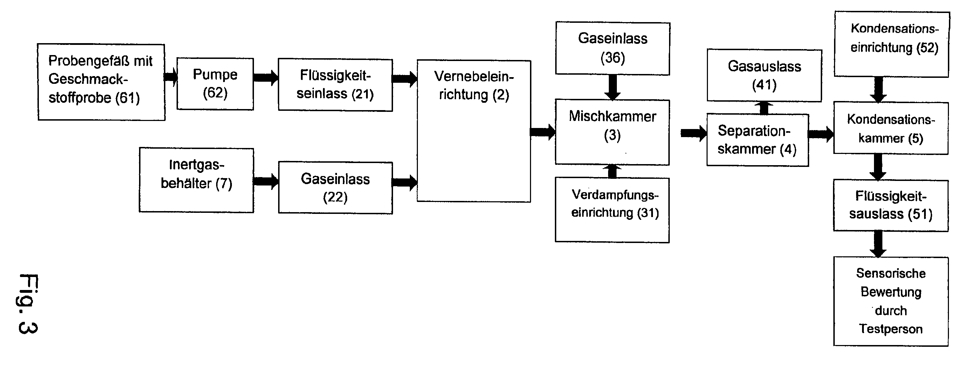

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass die Mischkammer (3) zusätzlich einen Gaseinlass (36) (siehe Figuren 2 und 3) zur direkten Zuführung eines Inertgases in die Mischkammer (3) umfasst. Diese bevorzugte Ausgestaltung ermöglicht es, die Strömungsgeschwindigkeit des Aerosols zusätzlich zu den Reglern des Flüssigkeitseinlasses (21) und des Gaseinlasses (22) zu regulieren.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass die Verdampfungseinrichtung (31) als Temperaturregler im Bereich von 30 bis 100 °C, vorzugsweise 50 bis 95 °C, weiter bevorzugt 70 bis 90 °C, besonders bevorzugt 75 bis 85 °C und ganz besonders von 80 °C ausgestaltet ist. Die Temperatur wird üblicherweise so eingestellt, dass eine ausreichende Menge an Ethanol in die Gasphase überführt wird, so dass der in der Separationskammer (4) aus der Vorrichtung (1) entfernte Ethanolanteil ausreicht, so dass das aus der Kondensationskammer (5) mittels des Flüssigkeitsauslasses (51) entfernte Kondensat einen Ethanolgehalt von ≤ 15 Gew.-%, bevorzugt im Bereich von 0 bis 10 Gew.-%, weiter bevorzugt im Bereich von 0,1 bis 8 Gew.-%, besonders bevorzugt im Bereich von 1 bis 5 Gew.-%, jeweils bezogen auf das Gesamtgewicht des Kondensats aufweist. Vorzugsweise liegt deshalb der Ethanolanteil, der in der Mischkammer (3) in die Gasphase übergeht, bei ≤ 50 Gew.-%, weiter bevorzugt im Bereich von 60 bis 100 Gew.-%, besonders bevorzugt im Bereich von 70 bis 95 Gew.-%, jeweils bezogen auf das Gesamtgewicht des der zugeführten Geschmackstoffzubereitung.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass die Mischkammer (3) als Röhre, d.h. mit einem (im wesentlichen) kreisförmigen Querschnitt von üblicherweise 50 mm, bevorzugt 35 mm und besonders bevorzugt 25mm und mit eine Länge von üblicherweise 150 mm, bevorzugt 120 mm und besonders bevorzugt 90 mm, ausgestaltet ist. Besonders bevorzugt weist diese Röhre einen gleichmäßigen Querschnitt auf und/oder ist nicht gekrümmt. Diese bevorzugte Ausgestaltung ist von Vorteil, da auf Grund der Röhrenform das Totvolumen minimiert wird und so zu einer turbulenzarmen Strömung führt.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass die Mischkammer (3) als doppelwandige Röhre, die um ihre erste innere Wand (32) eine dazu beabstandete zweite äußere Wand (33) mit einem Einlass (34) und einem Auslass (35) für ein Mittel zur Temperaturreglung aufweist, ausgestaltet ist. In dieser bevorzugten Ausgestaltung besteht die Verdampfungseinrichtung (31) in der doppelwandigen Röhrenausgestaltung (Heizmantel), bei der das Mittel zu Temperaturregelung, üblicherweise eine Flüssigkeit, bevorzugt eine Flüssigkeit ausgewählt aus der Gruppe bestehend aus Wasser oder Mineralöl als Wärmeübertragungsmittel und Wasser, Ethylenglykol oder Mischungen aus Wasser und Ethylenglykol als Kälteübertragungsmittel, auf die gewünschte Temperatur erwärmt und durch den Einlass (34) in den Raum zwischen der ersten inneren Wand (32) und der dazu beabstandeten zweiten äußeren Wand (33) zugeführt wird und aus dem Auslass (35) gegebenenfalls entfernt wird. Diese bevorzugte Ausgestaltung ermöglicht, dass die Mischkammer (3) (im wesentlichen) konstant temperiert werden kann, da immer neues temperiertes Mittel zur Temperaturregelung dem Heizmantel zugeführt wird bzw. bereits verwendetes Mittel zur Temperaturregelung nach Austritt aus dem Auslass (35) wieder auf die Ausgangstemperatur erwärmt werden kann.

In einer alternativen Ausgestaltung kann eine erfindungsgemäß zu verwendende Verdampfungseinrichtung eine Thermoelektrische Platte (Peltier Element), ein elektrisches Heizband, ein elektrischer Heizmantel oder ein elektrisches Heizrohr sein.

Die erfindungsgemäße Vorrichtung (1) ist zudem dadurch gekennzeichnet, dass die Separationskammer (4) einen größeren Querschnitt, d.h. (im wesentlichen) kreisförmigen Querschnitt, im Vergleich zu Mischkammer (3) umfasst, wobei der Querschnitt der Separationskammer (4) üblicherweise im Bereich von 100 mm, bevorzugt, 70 mm, besonders bevorzugt 50 mm und die Länge der Separationskammer (4) üblicherweise im Bereich von 150 mm, bevorzugt 100 mm und besonders bevorzugt 65 mm liegt. In einer ganz besonders bevorzugten erfindungsgemäßen Ausgestaltung ist die Separationskammer (4) kugelförmig ausgestaltet. Eine Veränderung des Querschnitts führt üblicherweise zu einer Diffusion des verdampften Aerosols, so dass Strömungsturbulenzen entstehen und eine Verwirbelung in der Separationskammer (4) unterstützt wird. Hierdurch wird die Separierung des Gasanteils und des flüssigen Aerosolanteils ebenfalls unterstützt, so dass der gasförmige Ethanolanteil verbessert durch den Gasauslass (41) aus der Separationskammer und damit aus der erfindungsgemäßen Vorrichtung (1) entfernt werden kann. In einer weiteren bevorzugten Ausgestaltung wird die Verwirbelung des Aerosols in der Separationskammer (4) weiter unterstützt durch ein oder mehrere Verwirbelungseinrichtungen (42), bevorzugt stab- oder dornähnliche Einrichtungen, die mit der Separationskammerwand (43) verbunden sind und sich in das innere der Separationskammer (4) erstrecken.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass der Gasauslass (41) als Röhre, d.h. mit einem (im wesentlichen) kreisförmigen Querschnitt, die durch eine äußere Wand (43) der Separationskammer (4) in einen Raum außerhalb der Vorrichtung (1) verläuft, ausgestaltet ist, wobei ein Ende der Röhre (411) innerhalb der Separationskammer (4) in einem Bereich, der gegenüber der Verbindung zur Mischkammer (3) liegt, und ein zweites Ende der Röhre (412) im Raum außerhalb der Vorrichtung (1) angeordnet ist. In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass der Gasauslass (41) im aufgebauten Zustand am unteren Ende der Separationskammer (5) angeordnet ist und in einem Bereich oberhalb des unteren Endes durch die äußere Wand (43) der Separationskammer (4) in den Raum außerhalb der Vorrichtung (1) verläuft.

Es ist bevorzugt, dass der Gasauslass (41) am unteren Ende der Separationskammer (4), d.h. in örtlicher Nähe zur Kondensationskammer (5) angeordnet ist, da durch das Temperaturgefälle die Flüssigkeitströpfchen des Aerosols überwiegend kondensieren und durch den Flüssigkeitsauslass (51) als Kondensat abgeführt werden können, während der überwiegend separierte Gasanteil des Aerosols durch den Gasauslass (41) aus der Vorrichtung (1) entfernt werden kann.

Der Gasauslass (41) ist bevorzugt eine Glasröhre, mit einem Querschnitt von 5 bis 15 mm, weiter bevorzugt ungefähr 10 mm und ist üblicherweise weniger als 10 mm, bevorzugt weniger als 5 mm, ganz besonders bevorzugt ungefähr 3 mm oberhalb des Übergangs zur Kondensationskammer (5) angeordnet. In einer weiteren bevorzugten Ausgestaltung ist das Ende der Röhre (411) trichterförmig ausgestaltet. Durch die trichterförmige Ausgestaltung der Öffnung (411) werden Turbulenzen in der Kondensationszone vermieden, so dass das Kondensat aus dem Flüssigkeitsauslass (51) gleichmäßig tropfen kann.

In einer bevorzugten Ausgestaltung ist der Gasauslass nicht linear sondern gewinkelt, bevorzugt in einem 90° Winkel angeordnet. Hierbei sind üblicherweise die Längen des Gasauslassrohres (41) senkrecht (Senkrechtröhre) und horizontal (Querröhre) zum Erdmittepunkt ungefähr gleichlang, bevorzugt ist die Senkrechtröhre ungefähr 27 mm lang und hat einen Abstand von 3 mm zur Kondensationskammer (5) und die Querröhre ist bevorzugt 30 mm lang.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass die Separationskammer (4) bei Umgebungstemperatur betrieben wird und nicht entsprechend der Kondensationskammer (5) gekühlt wird. Diese Ausgestaltung ist deshalb bevorzugt, weil durch die Kopplung der beheizten Mischkammer (3) und der gekühlten Kondensationskammer (5) über die erfindungsgemäß zu verwendende Separationskammer (4) ein "Temperaturpuffer" zwischen geschaltet wird, der Materialbeschädigungen an der Vorrichtung (1) auf Grund des Temperaturgefälles reduziert.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass die Kondensationskammer (5) eine Röhre, d.h. mit (im wesentlichen) kreisförmigen Querschnitt darstellt. Wie schon zur Mischkammer (3) oben ausgeführt weist eine Röhre ein geringes Totvolumen auf.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass die Röhre der Kondensationskammer (5) einen im Vergleich zur Separationskammer (4) und/oder der Mischkammer (3) kleineren Querschnitt hat, d.h. üblicherweise einen Querschnitt im Bereich von 8 mm, bevorzugt 5 mm und besonders bevorzugt 2 mm und eine Länge von üblicherweise 80 mm, bevorzugt 50mm und besonders bevorzugt 35 mm aufweist. Diese Querschnittsverringerung führt zu einer Kontraktion des in der Separationskammer (4) erhaltenen und in die Kondensationskammer (5) weitertransportierten Aerosols, so dass die Wirkung der Kondensationseinrichtung (52) unterstützt wird.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass die Kondensationseinrichtung (52) als Temperaturregler im Bereich von 0 bis 60 °C, vorzugsweise 5 bis 50 °C, weiter bevorzugt 10 bis 40 °C, besonders bevorzugt 15 bis 25 °C und ganz besonders von 20 °C ausgestaltet ist. Auf Grund dieser Temperaturbereiche wird die Kondensation des Aerosols zu dem erfindungsgemäßen Geschmackstoffkonzentrat unterstützt.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass die Kondensationskammer (5) als doppelwandige Röhre, die um ihre erste innere Wand (53) eine dazu beabstandete zweite äußere Wand (54) mit einem Einlass (55) und einem Auslass (56) für ein Mittel zur Temperaturreglung, üblicherweise eine Flüssigkeit, bevorzugt eine Flüssigkeit ausgewählt aus der Gruppe bestehend aus Wasser oder Mineralöl als Wärmeübertragungsmittel und Wasser, Ethylenglykol oder Mischungen aus Wasser und Ethylenglykol als Kälteübertragungsmittel, aufweist, ausgestaltet ist. Wie bereits zur erfindungsgemäßen Mischkammer (3) oben ausgeführt, ermöglicht diese bevorzugte Ausgestaltung, dass die Kondensationskammer (5) (im wesentlichen) konstant temperiert wird.

In einer alternativen Ausgestaltung kann eine erfindungsgemäß zu verwendende Kondensationseinrichtung (52) eine Thermoelektrische Platte (Peltier Element), ein elektrisches Temperierband, ein elektrischer Temperiermantel oder ein elektrisches Temperierrohr sein.

In einer weiteren bevorzugten Ausgestaltung ist die erfindungsgemäße Vorrichtung (1) zusätzlich oder alternativ zu den vorstehenden bevorzugten Ausgestaltungen dadurch gekennzeichnet, dass der Flüssigkeitsauslass (51) am unteren Ende der Kondensationskammer (5) angeordnet ist. Diese bevorzugte Ausgestaltung ermöglicht, dass das gebildete Kondensat mittels der Schwerkraft durch den Flüssigkeitsauslass (51) aus der Kondensationskammer (5) und damit aus der erfindungsgemäßen Vorrichtung (1) entfernt werden kann. Zusätzlich könnten allerdings auch weitere Mittel, insbesondere Pumpen (z.B. geeignete Schlauchpumpen oder LC-Pumpen - nicht gezeigt) der Kondensationskammer (5) nachgeschaltet werden, um das Kondensat aus der Vorrichtung (1) zu entfernen.

Die erfindungsgemäße Vorrichtung (1) kann aus jedem geeigneten Material, bevorzugt aus einem oder mehreren Materialien aus der Gruppe bestehend aus Glas, Metall, Edelmetall, Stahl oder Kunststoff (z.B. Polytetrafluorethylen oder Polyetheretherketon), besonders bevorzugt Glas hergestellt werden, wobei die einzelnen Kammern und Einrichtungen so mit einander verbunden sind, so dass aus der erfindungsgemäßen Vorrichtung (1) außer an den vorgesehenen Auslassstellen, insbesondere dem Gas - auslass (41) und dem Flüssigkeitsauslass (51), (im wesentlichen) weder das zugeführte Inertgas und/oder die zugeführte flüssige Zubereitung, bevorzugt die flüssige Geschmackstoffzubereitung aus der erfindungsgemäßen Vorrichtung (1) entweicht. In einer besonders bevorzugten Ausgestaltung ist die erfindungsgemäße Vorrichtung (1) zusätzlich oder alternativ zu den vorstehenden besonders bevorzugten Ausgestaltungen zum Teil oder vollständig aus wärmebeständigem Glas aufgebaut.

In einer weiteren bevorzugten Ausgestaltung kann zusätzlich oder alternativ zu den vorstehenden bevorzugten Ausgestaltungen das aus der erfindungsgemäßen Vorrichtung (1) entfernte Kondensat in zwei, drei oder mehreren Eluatfraktionen separiert werden, wobei die Fraktionen besonders bevorzugt derart separiert werden, dass eine Fraktion im wesentlichen einen Geschmackstoff umfasst. Zur Ermittlung einer Geschmackstoffkonzentratfraktion, die nur einen Geschmackstoff enthält, können ein oder mehrere, gleiche oder unterschiedliche geeignete Detektoren (nicht gezeigt), bevorzugt ausgewählt aus der Gruppe bestehend aus Massenselektiven Detektoren (MS), Ultraviolett Detektoren (UV), Diodenarray Detektoren (DAD), Brechungsindex-Detektoren (RI), Verdampfungslichtstreu Detektoren (ELSD - evaporative light scattering detector), Fluoreszenz Detektoren (FLD) oder Charged Aerosol Detektoren (CAD), die vor und/oder nach der erfindungsgemäßen Vorrichtung (1) angeordnet sind, verwendet werden. Diese bevorzugte Ausgestaltung ermöglicht die sensorische Bewertung des isolierten Geschmackstoffes und damit die Einschätzung des sensorischen Beitrags des Geschmackstoffes in einer Aromazubereitung.

Figur 2 zeigt einen schematischen Aufbau einer erfindungsgemäßen Vorrichtung (1), die ebenfalls die vorstehend dargestellten bevorzugten Ausgestaltungen in allen denkbaren Kombinationen umfasst, gekoppelt an ein erfindungsgemäß zu verwendendes Flüssigchromatographiesystem (6).

Die bevorzugte Ausgestaltung der erfindungsgemäße Vorrichtung (1) kann in einer bevorzugten Ausgestaltung mit den erfindungsgemäß verwendbaren Flüssigkeitschromatographiesystemen (6) unterschiedlicher Separationstechniken direkt, d.h. über den Flüssigkeitseinlass (21), verbunden werden, so dass das Eluat durch den Flüssigkeitseinlass (21) in die erste Vernebeleinrichtung (2) zugeführt werden kann.

Die Koppelung der erfindungsgemäßen Vorrichtung (1) ist zum einen deshalb bevorzugt, weil durch die flüssigchromatographische Auftrennung einer flüssigen Geschmackstoffprobe, die zwei, drei oder mehrere Geschmackstoffe umfasst, bereits eine zeitliche Auftrennung der Geschmackstoffe durch die eingesetzten Elutionsmittel Wasser und Ethanol, gegebenenfalls im Gradientenbetrieb vorgenommen wird, wobei das Eluat aus einer flüssigchromatographischen Auftrennung der erfindungsgemäßen Geschmackstoffzubereitung entspricht. Zum anderen umfasst das erfindungsgemäß zu verwendende Flüssigkeitschromatographiesystem (6) bereits eine Detektoreinrichtung die der erfindungsgemäßen Vorrichtung (1) dementsprechend vorgeschaltet ist, so dass eine bessere Abschätzung ermöglicht wird, wann der oder die Geschmackstoffe nach Durchlauf durch die erfindungsgemäße Vorrichtung (1) in dem hergestellten Geschmackstoffkonzentrat zu erwarten sind.

Durch die kompakte Bauform der erfindungsgemäßen Vorrichtung (1) und bevorzugt bei zusätzlicher Zuführung von Inertgas direkt in die Mischkammer (3) durch einen Gaseinlass (36) wird die Fließgeschwindigkeit eines LC-Eluats als flüssige Geschmackstoffzubereitung bzw. die Fließgeschwindigkeit des Aerosols, d.h. die Dauer des Transports durch die erfindungsgemäße Vorrichtung (1) so eingestellt, dass die flüssigchromatographische Trennleistung gewährleistet bleibt und so eine zeitnahe ("on-line") bzw. synchrone sensorische Bewertung hinsichtlich des Geschmackes eines einzigen Geschmackstoffes und damit des sensorischen Beitrags des Geschmackstoffes durch eine Testperson ermöglicht wird. Üblicherweise beträgt bei einem Druck von 3 bar an der Vernebeleinrichtung (2) die Aerosol/Gasgeschwindigkeit ~65ml/s. Die Passagedauer des Aerosols variiert in Abhängigkeit zu den Temperaturen (Verdampfung/Kondensation) in der Vorrichtung (1), zwischen Bruchteilen von Sekunden bis 1, 2 oder wenige Sekunden.

Figur 3 zeigt einen schematischen Aufbau einer erfindungsgemäßen Vorrichtung (1), die ebenfalls die vorstehend dargestellten bevorzugten Ausgestaltungen in allen denkbaren Kombinationen umfasst, gekoppelt an ein Probengefäß (61) enthaltend eine erfindungsgemäße Geschmackstoffzubereitung mit bevorzugt einem Geschmackstoff, die so miteinander verbunden sind, dass diese Geschmackstoffzubereitung gegebenenfalls mittels einer geeigneten üblichen Pumpe (62), beispielsweise LC-Pumpe oder Schlauchpumpe, durch den Flüssigkeitseinlass (21) in die erste Vernebeleinrichtung (2) zugeführt wird. Zwischen dem Probengefäß (61) und dem Flüssigkeitseinlass (21) kann bevorzugt eine Detektoreinrichtung (8) zwischengeschaltet sein, um besser beurteilen zu können, wann der oder die Geschmackstoffe durch die erfindungsgemäβe Vorrichtung (1) durchgeleitet werden.

Üblicherweise werden die Detektoren ausgewählt aus der Gruppe bestehend aus Massenselektiven Detektoren (MS), Ultraviolett Detektoren (UV), Diodenarray Detektoren (DAD), Brechungsindex-Detektoren (RI), Verdampfungslichtstreu Detektoren (ELSD - evaporative light scattering detector), Fluoreszenz Detektoren (FLD) oder Charged Aerosol Detektoren (CAD).

### Ausführunctsbeispiel:

Die vorliegende Erfindung wird im Folgenden durch ein Ausführungsbeispiel beschrieben, das jedoch den Schutzumfang der erfindungsgemäßen Gegenstände nicht limitiert.

### Beispiel 1: Identifizierung einer süßschmeckenden Verbindung aus Lippia dulcis

Ein Extrakt von Lippia dulcis (Aztekisches Süßkraut) wurde mittels Hochtemperatur Flüssigchromatographie (HTLC) unter Verwendung eines Wasser/ Ethanol-Gradienten separiert. Das Eluat wurde unter Verwendung des erfindungsgemäßen Verfahrens aufkonzentriert und sensorisch bewertet.

### 1. Herstellung einer Probelösung

Eine Probelösung Lippia dulcis wurde durch Lösung/Suspension von 500 mg Lippia dulcis in einem Gemisch Wasser/Ethanol (1:1 / v:v), mit anschließender Membranfiltration des nicht gelösten Rückstandes, hergestellt.

### 2. Durchführung einer Flüssigchromatographie der Probelösung

Die verwendete HTLC- Vorrichtung, bestehend aus einem Degasser (3315, Fa. ERC), zwei Pumpen (Sun Flow 100, Fa. Sunchrom), einem Mischer (dynamic/static Mixer, Fa. Sunchrom), einem Autosampler (Midas, Fa. Spark), einem Säulenofen (Polaratherm, Fa. Selerity) und einem Diodenarray-Detektor (Spectra Flow 600, Fa. Sunchrom) wurde mit einer Hamilton PRP-1 250x10mm Säule ausgestattet und mit einem Fluss von 2 ml/min eines Elutionsmittels, bestehend aus 50 Gew.-% Ethanol und 50 Gew.-% Wasser, konditioniert.

Zur Probenaufgabe in die verwendete, konditionierte HTLC-Vorrichtung wurden mit dem Autosampler 100µl der hergestellten klaren Lösung injiziert.

Nach erfolgter Konditionierung erfolgt die chromatographische Analyse nach folgendem Programm:

| Zeit [min] | Fluss [ml/min] | Wasser [%] | Ethanol [%] | Elutionsgradient [%/min] | Temperatur Säulenofen [°C] | Temperaturgradient [°C/min] | Diode array detector (DAD) |
|---|---|---|---|---|---|---|---|
| 0 | 2 | 50 | 50 | Isokratisch | 40 | isotherm | 210 nm |
| 6 | 2 | 50 | 50 | Isokratisch | 40 | 20 | 210 nm |
| 10 | 2 | 50 | 50 | 1,25 | 120 | isotherm | 210 nm |
| 50 | 2 | 0 | 100 | Isokratisch | 120 | isotherm | 210 nm |
| 60 | 2 | 0 | 100 | Isokratisch | 120 | isotherm | 210 nm |

Die Änderungen von Temperatur, Fluss und Elutionsmittel-Zusammensetzung während des Programms erfolgen linear.

Das Eluat wird im Anschluss an die chromatographische Trennung unter folgenden Bedingungen über den Flüssigkeitseinlass (21) direkt in die Vernebeleinrichtung (2) erfindungsgemäßen Vorrichtung (1) eingeleitet:

| Gasdruck im Gaseinlass (22) | Fluss im Flüssigkeitseinlass (21) | Temperatur Verdampfungseinrichtung (31) | Temperatur Kondensationsseinrichtung (52) |
|---|---|---|---|
| 3 bar | 2 ml/min | 80°C | 30°C |

Die in Lippia dulcis enthaltene sensorisch wirksame Verbindung Phyllodulcin eluierte nach flüssigchromatographischer Auftrennung bei einer Ethanolkonzentration von ca. 75%. Das Eluat wurde mit einem Druck von 3 bar über den Gaseinlass (22) in die erfindungsgemäße Vernebeleinrichtung (2) zugeführt und als vernebeltes Aerosol in die auf 80 C vorgeheizte Mischkammer (3) der erfindungsgemäßen Vorrichtung (1) zugeführt. Das am Flüssigkeitsauslass (51) der Kondensationskammer (5) gewonnene Kondensat bzw. erfindungsgemäßes Geschmackstoffkonzentrat wies einen Ethanolgehalt von ≤ 15% Ethanol auf.

Die erfindungsgemäßen Geschmackstoffkonzentrate der flüssigchromatographisch separierten Verbindungen wurden direkt durch ein Panel aus trainierten Testpersonen sensorisch bewertet. Phyllodulcin wurde durch einen typischen starken Süßgeschmack sensorisch nachgewiesen. Eine direkte sensorische Bewertung der flüssigchromatographisch separierten Verbindungen, ohne Verwendung der erfindungsgemäßen Vorrichtung und des erfindungsgemäßen Verfahrens hätte in dem beschriebenen Beispiel ein hohes Risiko der Fehleinschätzung des Geschmackseindruckes auf Grund der Beeinträchtigung der Geschmacksrezeptoren der Zunge durch den Ethanolgehalt beinhaltet und/oder eine Verkostung von mehreren Proben hintereinander zu einer zeitweisen Bewußtseinsbeeinträchtigung der Testpersonen ebenfalls auf Grund des Alkoholgehaltes geführt.

## Patentansprüche

1. Vorrichtung (1) zum Reduzieren eines Ethanolgehaltes in einer flüssigen Zubereitung mit einer Vernebeleinrichtung (2), einer Mischkammer (3), einer Separationskammer (4) und einer Kondensationskammer (5), **dadurch gekennzeichnet, dass**
- die Vernebeleinrichtung (2) einen Flüssigkeitseinlass (21), einen Gaseinlass (22) und eine Verbindung zur Mischkammer (3) umfasst oder daraus besteht und die Vemebeleinrichtung (2) bei einer Temperatur ≤ 100 °C betrieben wird, so dass eine Flüssigkeit umfassend Wasser und Ethanol mit einem Ethanolgehalt von > 15 Gew.-% bezogen auf das Gesamtgewicht der Flüssigkeit mittels des Flüssigkeitseinlasses (21) und ein Inertgas mittels des Gaseinlasses (22) der Vernebefeinrichtung (2) zuführbar sind und die Vernebeleinrichtung (2) geeignet ist, die Flüssigkeit und das Inertgas zu einem Aerosol zu vemebein und so in die Mischkammer (3) zuzuführen,
- die Mischkammer (3) eine Verdampfungseinrichtung (31) und eine Verbindung zur Separationskammer (4) umfasst oder daraus besteht, wobei die Mischkammer (3) nach der Vernebeleinrichtung (2) angeordnet und so damit verbunden ist, dass das mittels der Vernebeleinrichtung (2) gebildete Aerosol in die Mischkammer (3) zuführbar ist und die Verdampfungseinrichtung (31) geeignet ist, einen Teil oder das Ethanol des zugeführten Aerosols teilweise oder vollständig in die Gasphase zu überführen,
- die Separationskammer (4) einen Gasauslass (41) und eine Verbindung zur Kondensationskammer (5) umfasst oder daraus besteht, wobei die Separationskammer (4) nach der Mischkammer (3) angeordnet und so damit verbunden ist, dass das in die Gasphase überführte Ethanol und das verbleibende Aerosol In die Separationskammer (4) zuführbar sind und ein Teil der oder die Ethanol-Gasphase aus der Vorrichtung (1) durch den Gasauslass (41) entfernbar Ist und
- die Kondensationskammer (5) einen Flüssigkeitsauslass (51) und eine Kondensationseinrichtung (52) umfasst oder daraus besteht, wobei die Kondensationskammer (5) nach der Separationskammer (4) angeordnet und so damit verbunden ist, dass das in der Separationskammer (4) nach teilweiser oder vollständiger Entfernung der Ethanol-Gasphase verbleibende Aerosol in die Kondensationskammer (5) zuführbar ist und die Kondensationseinrichtung (5) geeignet ist, das zugeführte Aerosol ganz oder teilweise zu kondensieren und das Kondensat mit einem Ethanolgehalt von ≤ 15 Gew.-% bezogen auf das Gesamtgewicht des Kondensats aus der Kondensationskammer (5) durch den Flüssigkeitsauslass (51) entfernbar ist,
- wobei die Separationskammer (4) einen größeren Querschnitt im Vergleich zu Mischkammer (3) umfasst.

2. Vorrichtung gemäß Anspruch 1, **dadurch gekennzeichnet, dass** die Vorrichtung (1) im aufgebauten Zustand senkrecht zum Erdmittelpunkt aufgebaut ist und die Vernebeleinrichtung (2) am oberen Ende der Vorrichtung (1) und die Kondensationskammer (5) am unteren Ende der Vorrichtung (1) angeordnet sind.

3. Vorrichtung gemäß Anspruch 1 oder 2, **dadurch gekennzeichnet, dass** die Mischkammer (3) zusätzlich einen Gaseinlass (36) zur Zuführung eines Inertgases in die Mischkammer (3) umfasst.

4. Vorrichtung gemäß einem der vorstehenden Ansprüche, **dadurch gekennzeichnet, dass** die Mischkammer (3) als Röhre ausgestaltet ist.

5. Vorrichtung gemäß einem der vorstehenden Ansprüche, **dadurch gekennzeichnet, dass** der Gasauslass (41) als Röhre, die durch eine äußere Wand (43) der Separationskammer (4) in einen Raum außerhalb der Vorrichtung (1) verläuft, ausgestaltet ist, wobei ein Ende der Röhre (411) innerhalb der Separationskammer (4) in einem Bereich, der gegenüber der Verbindung zur Mischkammer (3) liegt, und ein zweites Ende der Röhre (412) im Raum außerhalb der Vorrichtung (1) angeordnet ist.

6. Vorrichtung gemäß Anspruch 5, **dadurch gekennzeichnet, dass** der Gasauslass (41) am im aufgebauten Zustand unteren Ende der Separationskammer (5) angeordnet ist und in einem Bereich oberhalb des unteren Endes durch die äußere Wand (43) der Separationskammer (4) in den Raum außerhalb der Vorrichtung (1) verläuft.

7. Vorrichtung gemäß einem der vorstehenden Ansprüche, **dadurch gekennzeichnet, dass** die Kondensationskammer (5) eine Röhre darstellt.

8. Vorrichtung gemäß einem der vorstehenden Ansprüche, **dadurch gekennzeichnet, dass** der Flüssigkeitsauslass (51) am unteren Ende der Vorrichtung (1) im aufgebauten Zustand angeordnet ist.

9. Verfahren zur Reduzierung eines Ethanolgehaltes in einer Geschmackstoffzubereitung umfassend oder bestehend aus ein oder mehreren Geschmackstoffen, Wasser und Ethanol umfassend oder bestehend aus den folgenden Schritten:
a) Bereitstellung einer flüssigen Geschmackstoffzubereitung, die ein oder mehrere Geschmackstoffe, Wasser und Ethanol mit einem Ethanolgehalt von 15 Gew.-% bezogen auf das Gesamtgewicht der Geschmackstoffzubereitung umfasst oder daraus besteht, sowie eines Inertgases,
b) Zuführung der flüssigen Geschmackstoffzubereitung durch einen Flüssigkeitseinlass (21) und des Inertgases durch einen Gaszugang (22) in eine Vernebeleinrichtung (2) mittels der die Geschmackstoffzubereitung und das Inertgas zu einem Aerosol vernebelt werden.
c) Zuführung des in Schritt b) gebildeten Aerosols in eine mit der Vernebeleinrichtung (2) verbundenen Mischkammer (3) mit Verdarnpfungseinrichtung (31) und Überführung eines Teils des oder des gesamten Ethanolanteils des Aerosols mittels der Verdampfungseinrichtung (31) in eine Gasphase,
d) Weiterleitung des in Schritt c) gebildeten Aerosols in eine mit der Mischkammer (3) verbundenen Separationskammer (4) mit Gasauslass (41) und Entfernung eines Teils des oder des gesamten gasförmigen Ethanolanteils aus der Separationskammer (4) durch den Gasauslass (41),
e) Weiterleitung des in Schritt d) gebildeten Aerosols in eine mit der Separationskammer (4) verbundene Kondensationskammer (5) mit Flüssigkeitsauslass (51) und Kondensationseinrichtung (52) und Kondensation des Aerosols mittels der Kondensationseinrichtung (52) zu einem Geschmackstoffkonzentrat umfassend oder bestehend aus dem oder den Geschmackstoffen, Wasser und Ethanol mit einem Ethanolgehalt von ≤ 15 Gew.-% bezogen auf das Gesamtgewicht des Geschmackstoffkonzentrats und
f) Entfernung des in Schritt e) gebildeten Geschmackstoffkonzentrats aus der Kondensationskammer (5) durch den Flüssigkeitsauslass (51),
wobei die Separationskammer (4) einen größeren Querschnitt im Vergleich zu Mischkammer (3) umfasst.

10. Verfahren gemäß Anspruch 9, **dadurch gekennzeichnet, dass** die Geschmackstoffzubereitung, die in Schritt a) der Vernebeleinrichtung (2) zugeführt wird ein mittels eines Flüssigchromatographiesystems (6) gewonnenes Eluat ist.

11. Verfahren gemäß Anspruch 10, **dadurch gekennzeichnet, dass** das Flüssigchromatographiesystem (6) mit dem Flüssigkeitseinlass (21) verbunden ist, so dass das Eluat direkt nach Auftrennung im Flüssigchromatographiesystem (6) durch den Flüssigkeitseinlass (21) in die Vernebeleinrichtung (2) zugeführt wird.

12. Verfahren zur sensorischen Bewertung von ein oder mehreren Geschmackstoffen umfassend oder bestehend aus den folgenden Schritten:
i. Herstellung eines flüssigen Geschmackstoffkonzentrates umfassend oder bestehend aus ein oder mehreren Geschmackstoffen, Wasser und Ethanol mit einem Ethanolgehalt von ≤ 15 Gew.-% bezogen auf das Gesamtgewicht des Geschmackstoffkonzentrats gemäß einem der Verfahren nach Ansprüchen 9 bis 10 und
ii. Zuführung des Geschmackstoffkonzentrates in den Mund einer Testperson zur sensorischen Bewertung.

13. Verfahren gemäß Anspruch 12, **dadurch gekennzeichnet, dass** die sensorische Bewertung gustatorisch und/oder retronasal durchgeführt wird.

14. Verwendung einer Vorrichtung (1) gemäß einem der Ansprüche 1 bis 8 zur Herstellung eines flüssigen Geschmackstoffkonzentrates umfassend oder bestehend aus ein oder mehreren Geschmackstoffen, Wasser und Ethanol mit einem Ethanolgehalt von ≤ 15 Gew.-% bezogen auf das Gesamtgewicht des Geschmackstoffkonzentrats.

## Claims

1. Device (1) for reducing an ethanol content in a liquid preparation with an atomising device (2), a mixing chamber (3), a separation chamber (4), and a condensation chamber (5), **characterised in that**
- the atomising device (2) comprises or consists of a liquid inlet (21), a gas inlet (22), and a connection to the mixing chamber (3), and the atomising device (2) is operated at a temperature of ≤ 100 °C, such that a liquid comprising water and ethanol, with an ethanol content of > 15% by weight in relation to the total weight of the liquid, can be fed by means of the liquid inlet (21) and an inert gas by means of the gas inlet (22) of the atomising device (2), and the atomising device (2) is suitable for atomising the liquid and the inert gas to an aerosol and so feeding it into the mixing chamber (3),
- the mixing chamber (3) comprises or consists of an evaporation device (31) and a connection to the separation chamber (4), wherein the mixing chamber (3) is arranged downstream of the atomising device (2) and is connected to it in such a way that the aerosol formed by means of the atomising device (2) can be fed into the mixing chamber (3) and the evaporation device (31) is suitable for transferring a part of the aerosol supplied, or the ethanol of the aerosol supplied, partially or completely into the gas phase,
- the separation chamber (4) comprises or consists of a gas outlet (41) and a connection to the condensation chamber (5), wherein the separation chamber (4) is arranged downstream of the mixing chamber (3) and is connected to it in such a way that the ethanol transferred into the gas phase and the remaining ethanol can be fed into the separation chamber (4) and a part of the or the ethanol gas phase, can be removed from the device (1) through the gas outlet (41), and
- the condensation chamber (5) comprises or consists of a liquid outlet (51) and a condensation device (52), wherein the condensation chamber (5) is arranged downstream of the separation chamber (4) and is connected to it in such a way that the aerosol remaining in the separation chamber (4) after partial or complete removal of the ethanol gas phase can be fed into the condensation chamber (5), and the condensation device (5) is suitable for condensing the aerosol which is fed completely or partially, and the condensate with an ethanol content of ≤ 15% by weight in relation to the total weight of condensate can be removed from the condensation chamber (5) through the liquid outlet (51),
- wherein the separation chamber (4) comprises a larger cross-section in comparison to the mixing chamber (3).

2. Device according to Claim 1, **characterised in that** the device (1) in the installed state is installed perpendicular to the centre of the earth, and the atomising device (2) is arranged at the upper end of the device (1) and the condensation chamber (5) at the lower end of the device (1).

3. Device according to Claim 1 or 2, **characterised in that** the mixing chamber (3) additionally comprises a gas inlet (36) for feeding an inert gas into the mixing chamber (3).

4. Device according to any one of the preceding Claims, **characterised in that** the mixing chamber (3) is designed as a tube.

5. Device according to any one of the preceding Claims, **characterised in that** the gas outlet (41) is designed as a tube, which runs through an outer wall (43) of the separation chamber (4) into a space outside the device (1), wherein one end of the tube (411) is arranged inside the separation chamber (4) in an area which is located opposite the connection to the mixing chamber (3), and a second end of the tube (412) is arranged in the space outside the device (1).

6. Device according to Claim 5, **characterised in that** the gas outlet (41) in the installed state is arranged at the lower end of the separation chamber (5), and runs in an area above the lower end through the outer wall (43) of the separation chamber (4) into the space outside the device (1).

7. Device according to any one of the preceding Claims, **characterised in that** the condensation chamber (5) takes the form of a tube.

8. Device according to any one of the preceding Claims, **characterised in that** the liquid outlet (51) in the installed state is arranged at the lower end of the device (1).

9. Method for reducing an ethanol content in a flavouring substance preparation, comprising or consisting of one or a plurality of flavouring substances, water, and ethanol, comprising or consisting of the following steps:
a) Preparing a liquid flavouring substance preparation, which comprises or consists of one or a plurality of flavouring substances, water, and ethanol, with an ethanol content of > 15% by weight in relation to the total weight of the flavouring substance preparation, and an inert gas,
b) Feeding the liquid flavouring substance preparation through a liquid inlet (21) and the inert gas through a gas inlet (22) into an atomising device (2) by means of which the flavouring substance preparation and the inert gas are atomised to an aerosol,
c) Feeding the aerosol formed in step b) into a mixing chamber (3) connected to the atomising device (2) with evaporation device (31), and transferring a part of or the total ethanol part of the aerosol into a gas phase by means of the evaporation device (31),
d) Transferring the aerosol formed in step c) into a separation chamber (4) connected to the mixing chamber (3) with a gas outlet (41), and removing a part of or the total of the gaseous ethanol part from the separation chamber (4) through the gas outlet (41),
e) Transferring the aerosol formed in step d) into a condensation chamber (5) connected to the separation chamber (4) with a liquid outlet (51) and condensation device (52), and condensing the aerosol by means of the condensation device (52) to a flavouring substance concentrate comprising or consisting of the flavouring substances, water, and ethanol, with an ethanol content of ≤ 15% by weight in relation to the total weight of the flavouring substance concentrate, and
f) Removing the flavouring substance concentrate formed in step e) from the condensation chamber (5) through the liquid outlet (51),
wherein the separation chamber (4) comprises a larger cross-section in comparison to the mixing chamber (3).

10. Method according to Claim 9, **characterised in that** the flavouring substance preparation which in step a) is fed into the atomising device (2) is an eluate obtained by means of a liquid chromatography system (6).

11. Method according to Claim 10, **characterised in that** the liquid chromatography system (6) is connected to the liquid inlet (21), such that the eluate is fed directly into the atomising device (2) through the liquid inlet (21) following separation in the liquid chromatography system (6).

12. Method for the sensory evaluation of one or a plurality of flavouring substances, comprising or consisting of the following steps:
i. Producing a liquid flavouring substance concentrate, comprising or consisting of one or a plurality of flavouring substances, water, and ethanol, with an ethanol content of ≤ 15% by weight in relation to the total weight of the favouring substance concentrate, according to any one of the methods according to Claims 9 to 10, and
ii. Feeding the flavouring substance concentrate into the mouth of a subject for sensory evaluation.

13. Method according to Claim 12, **characterised in that** the sensory evaluation is carried out using gustatory and/or retronasal perception.

14. Use of a device (1) according to any one of Claims 1 to 8 for producing a liquid flavouring substance concentrate, comprising or consisting of one or a plurality of flavouring substances, water, and ethanol, with an ethanol content of ≤ 15% by weight in relation to the total weight of the flavouring substance concentrate.

## Revendications

1. Dispositif (1) de réduction d'une teneur en éthanol d'une préparation liquide, comprenant un système de nébulisation (2), une chambre de mélange (3), une chambre de séparation (4) et une chambre de condensation (5), **caractérisé en ce que**
- le système de nébulisation (2) comprend ou se compose d'une arrivée de liquide (21), d'une arrivée de gaz (22) et d'une liaison avec la chambre de mélange (3) et le système de nébulisation (2) fonctionne à une température ≤ 100 °C de façon à pouvoir amener au système de nébulisation (2) un liquide comprenant de l'eau et de l'éthanol, avec une teneur en éthanol > 15 % en poids rapportée au poids total du liquide, par le biais de l'arrivée de liquide (21) et un gaz inerte par le biais de l'arrivée de gaz (22), et le système de nébulisation (2) est adapté pour nébuliser le liquide et le gaz inerte en un aérosol et pour l'introduire sous cette forme dans la chambre de mélange (3),
- la chambre de mélange (3) comprend ou se compose d'un système de vaporisation (31) et d'une liaison avec la chambre de séparation (4), la chambre de mélange (3) étant disposée après le système de nébulisation (2) et reliée à ce dernier de manière à pouvoir amener l'aérosol formé au moyen du système de nébulisation (2) dans la chambre de mélange (3), et le système de vaporisation (31) est adapté pour convertir en phase gazeuse tout ou partie de l'éthanol contenu dans l'aérosol introduit.
- la chambre de séparation (4) comprend ou se compose d'une sortie de gaz (41) et d'une liaison avec la chambre de condensation (5), la chambre de séparation (4) étant disposée après la chambre de mélange (3) et reliée à cette dernière de manière à pouvoir amener l'éthanol converti en phase gazeuse, et l'aérosol restant dans la chambre de séparation (4) et la phase éthanol gazeuse peut être évacuée en tout ou partie du dispositif (1) par le biais de la sortie de gaz (41), et
- la chambre de condensation (5) comprend ou se compose d'une sortie de liquide (51) et d'un système de condensation (52), la chambre de condensation (5) étant disposée après la chambre de séparation (4) et reliée à cette dernière de façon à pouvoir amener l'aérosol restant dans la chambre de séparation (4) après l'évacuation partielle ou totale de la phase éthanol gazeuse dans la chambre de condensation (5), et le système de condensation (5) est adapté pour condenser tout ou partie de l'aérosol introduit et le condensat, ayant une teneur en éthanol ≤ 15 % en poids rapportée au poids total du condensat, peut être évacué de la chambre de condensation (5) par le biais de la sortie de liquide (51),
- la chambre de séparation (4) présentant une aire de section plus grande que celle de la chambre de mélange (3).

2. Dispositif selon la revendication 1, **caractérisé en ce que**, à l'état monté, le dispositif (1) est placé perpendiculairement au centre de la Terre et **en ce que** le système de nébulisation (2) est disposé à l'extrémité supérieure du dispositif (1) et la chambre de condensation (5) est disposée à l'extrémité inférieure du dispositif (1).

3. Dispositif selon la revendication 1 ou la revendication 2, **caractérisé en ce que** la chambre de mélange (3) comprend en plus une arrivée de gaz (36) pour introduire un gaz inerte dans la chambre de mélange (3).

4. Dispositif selon l'une des revendications précédentes, **caractérisé en ce que** la chambre de mélange (3) est réalisée sous la forme d'un tube.

5. Dispositif selon l'une des revendications précédentes, **caractérisé en ce que** la sortie de gaz (41) est réalisée sous la forme d'un tube passant à travers une paroi extérieure (43) de la chambre de séparation (4) dans un espace à l'extérieur du dispositif (1), une extrémité du tube (411) étant disposée à l'intérieur de la chambre de séparation (4), dans une zone qui se trouve face à la liaison avec la chambre de mélange (3), et une deuxième extrémité du tube (412) étant disposée dans l'espace à l'extérieur du dispositif (1).

6. Dispositif selon la revendication 5, **caractérisé en ce que** la sortie de gaz (41) est disposée à l'extrémité inférieure, à l'état monté, de la chambre de séparation (5) et, dans une zone au-dessus de l'extrémité inférieure, passe à travers la paroi extérieure (43) de la chambre de séparation (4) dans l'espace à l'extérieur du dispositif (1).

7. Dispositif selon l'une des revendications précédentes, **caractérisé en ce que** la chambre de condensation (5) représente un tube.

8. Dispositif selon l'une des revendications précédentes, **caractérisé en ce que** la sortie de liquide (51) est disposée à l'extrémité inférieure du dispositif (1) à l'état monté.

9. Procédé de réduction d'une teneur en éthanol dans une préparation aromatisante comprenant ou se composant d'un ou plusieurs agents aromatisants, d'eau et d'éthanol, comprenant ou constitué par les étapes suivantes :
a) fourniture d'une préparation aromatisante liquide qui comprend ou se compose d'un ou plusieurs agents aromatisants, d'eau et d'éthanol, avec une teneur en éthanol > 15 % en poids rapportée au poids total de la préparation aromatisante, ainsi que d'un gaz inerte,
b) introduction de la préparation aromatisante liquide par une arrivée de liquide (21) et du gaz inerte par une arrivée de gaz (22) dans un système de nébulisation (2) au moyen duquel la préparation aromatisante et le gaz inerte sont nébulisés en un aérosol,
c) introduction de l'aérosol formé à l'étape b) dans une chambre de mélange (3) reliée au système de nébulisation (2) et comprenant un système de vaporisation (31), et conversion en phase gazeuse de tout ou partie de la fraction éthanolique de l'aérosol au moyen du système de vaporisation (31),
d) transfert de l'aérosol formé à l'étape c) dans une chambre de séparation (4) reliée à la chambre de mélange (3) et comprenant une sortie de gaz (41), et évacuation de tout ou partie de la fraction éthanolique gazeuse hors de la chambre de séparation (4) par le biais de la sortie de gaz (41),
e) transfert de l'aérosol formé à l'étape d) dans une chambre de condensation (5) reliée à la chambre de séparation (4) et comprenant une sortie de liquide (51) et un système de condensation (52), et condensation de l'aérosol au moyen du système de condensation (52) pour former un concentré aromatisant comprenant ou constitué du ou des agents aromatisants, d'eau et d'éthanol, avec une teneur en éthanol ≤ 15 % en poids rapportée au poids total du concentré d'agent aromatisant, et
f) évacuation du concentré d'agent aromatisant formé à l'étape e) hors de la chambre de condensation (5) par le biais de la sortie de liquide (51),
la chambre de séparation (4) présentant une aire de section plus grande que celle de la chambre de mélange (3).

10. Procédé selon la revendication 9, **caractérisé en ce que** la préparation aromatisante introduite dans le système de nébulisation (2) à l'étape a) est un éluat obtenu au moyen d'un système de chromatographie liquide (6).

11. Procédé selon la revendication 10, **caractérisé en ce que** le système de chromatographie liquide (6) est reliée à l'arrivée de liquide (21) de telle façon que l'éluat est introduit dans le système de nébulisation (2), par le biais de l'arrivée de liquide (21), directement après avoir été séparé dans le système de chromatographie liquide (6).

12. Procédé d'évaluation organoleptique d'un ou plusieurs agents aromatisants, comprenant ou constitué par les étapes suivantes :
i. préparation d'un concentré aromatisant liquide comprenant ou se composant d'un ou plusieurs agents aromatisants, d'eau et d'éthanol, avec une teneur en éthanol ≤ 15 % en poids rapportée au poids total du concentré aromatisant, par un des procédés suivant les revendications 9 à 10, et
ii. introduction du concentré aromatisant dans la bouche d'un sujet de test pour l'évaluation organoleptique.

13. Procédé selon la revendication 12, **caractérisé en ce que** l'évaluation organoleptique est une évaluation gustative et/ou rétronasale.

14. Utilisation d'un dispositif (1) selon l'une des revendications 1 à 8 pour préparer un concentré aromatisant liquide qui comprend ou se compose d'un ou plusieurs agents aromatisants, d'eau et d'éthanol, avec une teneur en éthanol ≤ 15 % en poids rapportée au poids total du concentré aromatisant.
